(19) Europäisches Patentamt / European Patent Office / Office européen des brevets

(11) **EP 3 888 753 B1**

(12) **EUROPEAN PATENT SPECIFICATION**

(45) Date of publication and mention
of the grant of the patent:
**04.03.2026 Bulletin 2026/10**

(21) Application number: **19890352.8**

(22) Date of filing: **26.11.2019**

(51) International Patent Classification (IPC):
*A61P 17/16* (2006.01)    *A61Q 19/00* (2006.01)
*A61K 9/12* (2006.01)    *A61K 8/02* (2006.01)
*A61K 8/34* (2006.01)    *A61K 8/63* (2006.01)
*A61K 8/81* (2006.01)    *A61K 8/92* (2006.01)
*A61K 47/10* (2017.01)    *A61K 47/24* (2006.01)
*A61K 47/28* (2006.01)    *A61K 47/32* (2006.01)
*A61K 47/44* (2017.01)    *A61K 8/87* (2006.01)
*A61K 8/891* (2006.01)    *A61K 9/70* (2006.01)
*A61L 26/00* (2006.01)    *A61K 8/86* (2006.01)

(52) Cooperative Patent Classification (CPC):
**A61L 26/0052; A61K 8/02; A61K 8/34;**
**A61K 8/8129; A61K 8/86; A61K 8/87; A61K 8/891;**
**A61K 9/7007; A61K 47/10; A61K 47/24;**
**A61K 47/28; A61K 47/32; A61K 47/44;**
**A61L 26/0076; A61P 17/16;**    (Cont.)

(86) International application number:
**PCT/JP2019/046152**

(87) International publication number:
**WO 2020/111055 (04.06.2020 Gazette 2020/23)**

(54) **COATING FILM FOR SKIN**

ÜBERZUGSFILM FÜR HAUT

FILM DE REVÊTEMENT POUR LA PEAU

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **26.11.2018 JP 2018220755**

(43) Date of publication of application:
**06.10.2021 Bulletin 2021/40**

(73) Proprietor: **Kao Corporation**
**Chuo-ku**
**Tokyo 103-8210 (JP)**

(72) Inventors:
• **SASAOKA, Shun**
  **Tokyo 131-8501 (JP)**
• **KIRINO, Aya**
  **Haga-gun, Tochigi 321-3497 (JP)**
• **SUZUKI, Hiroshi**
  **Tokyo 131-8501 (JP)**
• **NAGANO, Kenichi**
  **Tokyo 131-8501 (JP)**
• **GABE, Yu**
  **Odawara-shi, Kanagawa 250-0002 (JP)**
• **UCHIYAMA, Masayuki**
  **Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
JP-A- 2003 506 470    JP-A- 2004 002 356
JP-A- 2009 536 647    JP-A- 2012 017 297
JP-A- 2017 078 062    JP-A- 2018 087 186
JP-A- 2018 108 991

• **ANONYMOUS: "Relative permittivity - Wikipedia", 19 October 2023 (2023-10-19), pages 1 - 8, XP093136714, Retrieved from the Internet <URL:https://en.wikipedia.org/wiki/ Relative_permittivity> [retrieved on 20240301]**

(52) Cooperative Patent Classification (CPC): (Cont.)
**A61Q 19/00;** A61L 2400/12

**Description**

Field of the Invention

The present invention relates to a coating film for a skin, which is formed on a human skin, the coating film being a fiber layer comprising a fiber assembly composed of a nanofiber deposit and a liquid preparation and is as defined in the appended claims.

Background of the Invention

Various methods are known for forming a coating containing fibers by electrostatic spraying. For example, PTL 1 discloses a method using a portable electrospinning device, but there is no specific description of the solvents for injecting a liquefied polymer. PTL 2 discloses a method for forming a discontinuous film on the skin by electrostatic spraying, but the discontinuous film obtained is a liquid coating containing particles, but not a coating containing fibers. PTL 3 discloses a method for forming fibers by direct electrostatic spraying on humans. As a specific example, disclosed is a coating film formed by electrostatically spraying a composition in which polyvinyl alcohol is dissolved in a water-ethanol solution.

PTL1: JP-A-2004-525272

PTL2: JP-A-2006-95332

PTL3: JP-A-2007-325934

JP 2003-506470A relates to substantially uniform, discontinuous films of a skin care product having a defined average particle size, particle spacing and coverage value. The films are preferably formed by electrostatically spraying the composition onto the skin.

JP 2004-002356A concerns a color-changing cosmetic comprises one or more kinds of coloring agents, one or more kinds of developers and one or more color-changing agents.

JP 2009-536647A describes a dermal patch having comprising at least two layers wherein at least one layer is a polymer matrix system having an active agent admixed therein.

JP 2012-017297A relates to a stick cosmetic comprising a hydrocarbon wax and a liquid hydrocarbon oil.

JP 2017-078062A, JP 2018-087186A and JP2018-108991A describe cosmetic coatings for the skin, which are applied by electrostatic spraying of compositions comprising a film-forming polymer and a volatile solvent.

Summary of the Invention

The present invention relates to a coating film for human skin comprising a fiber layer, wherein the fiber layer comprises: a fiber assembly in which fiber of a water-insoluble polymer with a fiber diameter of 50 nm or more and less than 10,000 nm is deposited; and a liquid preparation X present between fibers and on a surface of fibers in the fiber assembly, the fiber assembly has a basis weight Q of 0.08 mg/cm$^2$ or more and 1.0 mg/cm$^2$ or less, and the liquid preparation X has a basis weight P of 0.5 mg/cm$^2$ or more and 2.3 mg/cm$^2$ or less and a relative permittivity of 2 or more and 27 or less, and comprises a component (a): oil agent in a liquid state at 20°C and has a relative permittivity of 0.5 or more and 20 or less, and optionally a component (b): polyol in a liquid state at 20°C, in which the content of the component (b) in the liquid preparation X is 50 mass% or less; wherein the ratio of the basis weight P of the liquid preparation X to the basis weight Q of the fiber assembly (P/Q) is 2.5 or more and 10 or less, wherein the determination of the relative permittivity of the liquid preparation X is described in this specification.

Another aspect of the present invention relates to a non-therapeutic method for producing the coating film.

Brief Description of the Drawings

Figure 1 is a schematic diagram showing how an electrostatic spraying method is carried out using an electrostatic spraying device.
Figure 2 is an outline diagram showing a configuration of an electrostatic spraying device which is suitably used in the present invention.

Detailed Description of the Embodiment

PTL 1 discloses a coating film formed from a liquefied polymer, and the coating film is composed only of a fiber layer, and therefore it needs further improvement for moderately and consistently suppressing water evaporation from the skin. PTL 2 discloses a coating film in which a liquid preparation is combined with a fiber layer to improve the adhesion of the fiber layer. However, the problem of moderately suppressing water evaporation from the skin, and specific methods are not described, and there is room for further investigation.

For the coating film disclosed in PTL 3, in order to spray the composition to the skin directly, the composition needs to be sprayed at high temperature to dry the composition. It follows that the spraying is done as a liquid droplet, and it is difficult to form a fibrous film. In addition, when a water-soluble fiber is formed, the fiber alone is formed, and there remains room for improvement in moderate suppression of water evaporation from the skin.

Accordingly, the present invention relates to: a coating film for human skin which is formed on the human skin, the coating film comprising a fiber assembly which is a fiber deposit and a liquid preparation present on a surface of fibers or between fibers, which results in the coating film with moderate suppression of water evaporation while maintaining a use impression in which a feeling of pressure on the skin is suppressed; and a method for producing the coating film for human skin.

The present inventors have energetically conducted studies on a combination of a fiber assembly composed of a fiber deposit and a liquid preparation present on a surface of fibers or between fibers, as a coating film which is formed on a skin surface and is capable of moderately suppressing water evaporation. As a result, it was found that a coating film containing a combination of a fiber assembly with a predetermined basis weight and a specific liquid preparation containing liquid oil is useful for securing both use impression and control of water evaporation. In this way, the present invention was attained.

The coating film for human skin in the present invention is excellent in use impression and capable of moderately suppressing water evaporation from the skin. Further, application of the coating film for human skin which moderately suppresses water evaporation enables improvement of skin metabolism.

The coating film or human skin in the present invention contains a fiber layer. The fiber layer contains a fiber assembly by deposition of fiber so called nanofiber having a fiber diameter of 50 nm or more and 10,000 nm or less.

The coating film for human skin of the present invention is a coating film formed directly on the skin. By discharging a composition B containing a component (e): volatile substance and a component (f): water-insoluble polymer as described later directly to the skin using an electrospinning device, an aerosol container or a pressure accumulation-type trigger spray container, nanofiber containing the component (f) and having the above-described fiber diameter can be formed.

In the present invention the nanofiber is formed by an electrospinning method from the viewpoint of more reliably forming nanofiber having the above-described fiber diameter.

The electrospinning method is a method in which a positive or negative high voltage is applied to the composition B to charge the composition B, and the charged composition B is discharged to the skin. The discharged composition B spreads into the space while being repeatedly micronized by a coulomb repulsive force, and in this process or after adherence of the composition B to the skin, the component (e) which is a volatile substance evaporates to form a fiber assembly by deposition of fiber on the skin surface.

The composition B for use in the present invention is in a liquid state in an environment where the electrospinning method is carried out. The composition B contains the following component (e) and component (f):

(e) one or more volatile substances selected from the group consisting of alcohols; and
(f) a water-insoluble polymer capable of forming a coating film.

Hereinafter, the components of the composition B will be described.

The alcohol as the component (e) is a volatile substance, which has volatility in a liquid state. In the composition (B), the component (e) is added for the purpose of ultimately forming a dry coating film on the skin through a process in which after the composition (B) placed in an electric field is sufficiently charged, the component (e) is discharged from a nozzle tip of the device to the skin, and when the component (e) is evaporated, the charge density of the composition B becomes excessive, and the component (e) further evaporates while being further micronized by coulomb repulsion. For this purpose, a monohydric chain aliphatic alcohol, a monohydric cyclic aliphatic alcohol or a monohydric aromatic alcohol is preferably used as the alcohol which is a volatile substance. Examples of the monohydric chain aliphatic alcohol include C1-C6 alcohols, examples of the monohydric cyclic aliphatic alcohol include C4-C6 cyclic alcohols, and examples of the monohydric aromatic alcohol include benzyl alcohol and phenylethyl alcohol. Specific examples thereof include ethanol, isopropyl alcohol, butyl alcohol, phenylethyl alcohol, n-propanol and n-pentanol, and from the viewpoint of fiber formability and feel impression, one or more selected from the group consisting of ethanol, isopropyl alcohol, butyl alcohol and n-propanol are preferable, at least ethanol is preferably contained, and ethanol is even more preferable. In the present invention, the volatile substance is one having a vapor pressure of 1.33 kPa or more and 40.00 kPa or less at 20°C.

The content of the component (e) in the composition B is preferably 45 mass% or more, more preferably 50 mass% or more, further more preferably 55 mass% or more, and preferably 95 mass% or less, more preferably 94 mass% or less, further more preferably 90 mass% or less, from the viewpoint of fiber formability by electrospinning. When the ethanol is contained, the content of the ethanol is preferably 50 mass% or more, more preferably 80 mass% or more, and preferably 100 mass% or less, and may be 100 mass% relative to the total amount of volatile substance of the component (e).

The composition B for forming fiber contains the component (e) as well as a water-insoluble polymer capable of forming a coating film, which is the component (f). The water-insoluble polymer capable of forming a coating film, which is the component (f), is typically a substance soluble in the volatile substance which is the component (e). Here, the term "soluble" means that the substance is in a dispersed state at 20°C, and the dispersed state is a visually homogeneous state, preferably a visually transparent or translucent state.

As the component (f): water-insoluble polymer capable of forming a coating film, appropriate one is used in accordance with the properties of the volatile substance which is the component (e). In the present description, the "water-insoluble polymer" is one having a property such that 1 g of the polymer is weighed in an environment at 1 atm and 23°C, and then is immersed in 10 g of ion-exchanged water, and after elapse of 24 hours, more than 0.5 g of the immersed polymer remains insoluble.

Examples of the component (f): water-insoluble polymer capable of forming a coating film include fully saponified polyvinyl alcohols capable of being subjected to insoluble treatment after formation of the coating film, partially saponified polyvinyl alcohols capable of being subjected to crosslinking treatment after formation of the coating film when used in combination with a crosslinker, oxazoline-modified silicones such as poly(N-propanoylethyleneimine)graft-dimethylsiloxane/y-aminopropylmethylsiloxane copolymers, polyvinylacetal diethylaminoacetate, Zein (main components of corn protein), polyester, polylactic acid (PLA), polyacrylonitrile resins, acrylic resins such as polymethacrylic acid resins, polystyrene resins, polyvinyl butyral resins, polyethylene terephthalate resins, polybutylene terephthalate resins, polyurethane resins, polyamide resins, polyimide resins, and polyamideimide resins. These water-insoluble polymers may be used alone (singly) or in combination of two or more thereof.

Of these water-insoluble polymers, fully saponified polyvinyl alcohols capable of being subjected to insoluble treatment after formation of the coating film, partially saponified polyvinyl alcohols capable of being subjected to crosslinking treatment after formation of the coating film when used in combination with a crosslinker, polyvinyl butyral resins, acrylic resins such as (alkyl acrylate-octylamide) copolymers, oxazoline-modified silicones such as poly(N-propanoylethyleneimine)graft-dimethylsiloxane/y-aminopropylmethylsiloxane copolymers, polyurethane resins, polyester, Zein and the like are preferably used.

The content of the component (f) in the composition B for forming the fiber assembly is preferably 5 mass% or more, more preferably 7 mass% or more from the viewpoint of fiber formability, and preferably 35 mass% or less, more preferably 30 mass% or less from the viewpoint of preventing deterioration of the discharge property due to elevation of the viscosity of the composition B by the water-insoluble polymer. The optimum content of the component (f) is appropriately adjusted in accordance with the properties of the component (f) and conditions during the discharge.

The composition B for forming the fiber assembly may further contain various components such as water, an oil agent in a liquid state at 20°C and a polyol in a liquid state at 20°C as long as fiber formation is not hampered. The amount of water in the composition B is preferably 8 mass% or less, more preferably 5 mass% or less, further more preferably 2 mass% or less from the viewpoint of fiber formability.

The water, the oil agent, the polyol and the like contained in the composition B may be contained in the fiber during formation of the fiber assembly, to be formed, and in the present invention, they are contained in a liquid preparation X present between fibers and on the surface of fibers in the fiber assembly regardless of whether or not bleed-out occurs. Therefore, the liquid preparation X in the present invention is a liquid component left after removal of volatile components such as alcohol and the water-insoluble polymer from an external composition A and the composition B.

In the composition B, from the viewpoint of fiber formability, the mass ratio of the oil agent in a liquid state at 20°C and the polyol in a liquid state at 20°C to the component (f), (oil agent + polyol) / [component (f)], is preferably 3 or less, more preferably 2.5 or less, further more preferably 2 or less, and preferably 0 or more, more preferably 0.2 or more.

When a fiber assembly composed of a fiber deposit is formed by an electrospinning method or the like, the thickness of the fiber (fiber diameter) is 50 nm or more, preferably 100 nm or more, more preferably 200 nm or more, and 10,000 nm or less, preferably 2,000 nm or less, more preferably 1,000 nm or less, in terms of an equivalent circle diameter. The thickness of the fiber can be measured by, for example, observing the fiber at a magnification of 10,000 times with a scanning electron microscope (SEM), removing defects (fiber lumps, fiber cross-points, and droplets) from two dimensional images, randomly selecting 10 fibers, drawing a line orthogonal to the longitudinal direction of the fiber, and directly reading the fiber diameter.

The fiber in the fiber assembly of the present invention is preferably continuous fiber, and the continuous fiber has a length at least 100 times as much as the thickness of the fiber. It is preferable that for example, the fiber in a fiber absorber comprise fiber comprising the component (f) and having a length of preferably 10 $\mu$m or more, more preferably 50 $\mu$m or more, further more preferably 100 $\mu$m or more. The cross-sectional shape of the fiber is preferably a circular shape or an elliptic shape, and the thickness of the fiber is the diameter of the circular shape, or the length of the major axis of the elliptic shape. The fiber assembly is a coating film which is composed of a deposit of one or more continuous fibers, and has fiber cross-points, but is generally porous and discontinuous, and the assembly forms one sheet. Fibers may closely adhere at the fiber cross-points.

The basis weight Q of the fiber assembly of the present invention is a basis weight of solid contents of the water-insoluble polymer forming the fiber, and can be determined from the mass of the water-insoluble polymer in the composition B discharged for forming the fiber and the area of the fiber assembly formed by discharging the composition B. The basis weight is 0.08 mg/cm$^2$ or more, preferably 0.1 mg/cm$^2$ or more, more preferably 0.12 mg/cm$^2$ or more from the viewpoint of securing a stable vapor permeability of the coating film, and 1.0 mg/cm$^2$ or less, preferably 0.8 mg/cm$^2$ or less, more preferably 0.5 mg/cm$^2$ or less from the viewpoint of further suppressing a feeling of pressure.

The electrospinning method in the present invention may be carried out by using an electrostatic spraying device. Specifically, it is preferable to directly discharge the composition B onto the skin by using a hand-held type electrostatic spraying device whose main body or operation unit equipped with a discharge nozzle is holdable with one hand to form a fiber assembly.

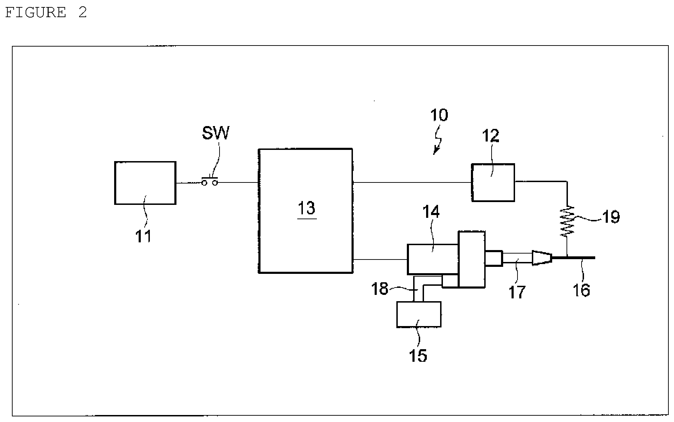

The electrostatic spraying device has a container accommodating the composition B for electrospinning, a nozzle for discharging the composition B, a supply device for supplying the composition B accommodated in the container to the nozzle, and a power source for applying a voltage to the nozzle. Figure 2 shows a schematic diagram indicating an electrostatic spraying device preferably used in the present invention. An electrostatic spraying device 10 shown in Figure 2 has a low-voltage power source 11. The low- voltage power source 11 is one which can generate a voltage of several volts to several tens of volts. For the purpose of enhancing transportability of the electrostatic spraying device 10, it is preferable that the low-voltage power source 11 be composed of one or more batteries. Use of batteries as the low-voltage power source 11 exhibits also such an advantage that the batteries can be exchanged easily as required. In place of the batteries, an AC adaptor or the like can be used as the low-voltage power source 11.

The electrostatic spraying device 10 has also a high-voltage power source 12. The high-voltage power source 12 is connected to the low-voltage power source 11, and has an electronic circuit (not shown in figure) for boosting a voltage generated by the low-voltage power source 11 to a high voltage. The boosting electronic circuit is usually constituted of a transformer, capacitors, semiconductor elements and the like.

The electrostatic spraying device 10 further has an auxiliary electrical circuit 13. The auxiliary electric circuit 13 is interposed between the low-voltage power supply 11 and the high-voltage power supply 12, and has a function of adjusting the voltage of the low-voltage power supply 11 to stably operate the high-voltage power supply 12. Further, the auxiliary electric circuit 13 has a function of controlling the number of revolutions of a motor provided in a piston pump 14 or a microgear pump 14. By controlling the rotation speed of the motor, the electrostatic spraying device serves as a supplying device for supplying the composition B accommodated in the container to the nozzle.

A switch SW is mounted between the auxiliary electric circuit 13 and the low-voltage power supply 11 such that the electrostatic spraying device 10 can be operated/suspended by turning the switch SW on and off.

The electrostatic spraying device 10 further has a nozzle 16. The nozzle 16 is composed of various electroconductors including metals and non-electroconductors such as plastics, rubbers and ceramics, and has a shape which can discharge the composition B from its tip. In the nozzle 16, a fine space through which the composition B flows is formed in the longitudinal direction of the nozzle 16. It is preferable that the size of the fine space for the size of the cross section be 100 $\mu$m or more and 1,000 $\mu$m or less in diameter. The nozzle 16 communicates with the microgear pump 14 or the piston pump 14 through a pipe 17. The pipe 17 may be an electroconductor or a non-electroconductor. The nozzle 16 is electrically connected to the high-voltage power source 12. Thereby, the nozzle 16 is so configured that a high voltage can be applied thereto. In this case, in order to prevent an excessive current from flowing in a human body when the human body touches directly the nozzle 16, the nozzle 16 and the high-voltage power source 12 are electrically connected through a current limiting resistance 19.

The microgear pump 14 or the piston pump 14 which communicate with the nozzle 16 via the pipe 17 functions as a supply device for supplying the nozzle 16 with the composition B contained in the container 15. The microgear pump 14 or the piston pump 14 are operated by receiving a power supply from the low-voltage power supply 11. The microgear pump 14 or the piston pump 14 are configured to supply a predetermined amount of the composition B to the nozzle 16 under the control of the auxiliary electric circuit 13.

It is preferable that the container 15 containing the composition B have a cartridge-type exchangeable form.

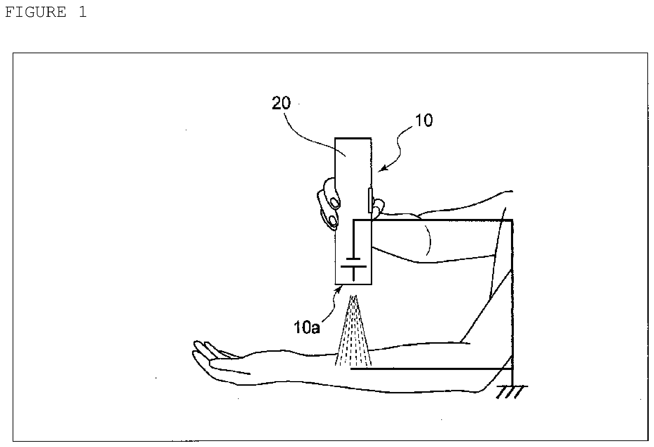

The electrostatic spraying device 10 having the above configuration can be used, for example, as shown in FIG. 1. FIG. 1 shows an electrostatic spraying device 10 of the hand-held type having a size holdable by one hand. The electrostatic spraying device 10 shown in the figure contains all of the members of the configuration diagram shown in Fig. 2 in the cylindrical housing 20. A nozzle, not shown, is disposed at one longitudinal end 10a of the housing 20. The nozzle is disposed in the housing 20 such that the blowing direction of the composition coincides with the longitudinal direction of the housing 20 and the nozzle is protruding toward the skin side, which is the coating formation object.

By arranging the nozzle tip so as to protrude toward the coating formation object in the longitudinal direction of the housing 20, the composition B is hardly adhered to the housing, and the coating can be stably formed.

When a user of the electrostatic spraying device 10 operates the electrostatic spraying device 10 for forming the fiber assembly on his or her skin, the user, **i.e. a** person who forms the coating film on his or her skin by electrostatic spraying, grasps the device 10 with hands, and directs one end 10a of the device 10, at which a nozzle (not shown) is disposed, to a target part on which electrostatic spraying is performed. Figure 1 shows a state in which one end 10a of the electrostatic spraying device 10 is directed to the inner side of the forearm region of the user. Under the state, the device 10 is turned on to carry out the electrostatic spraying method. When the device 10 is powered on, an electric field is generated between the nozzle and the skin. In the embodiment shown in Figure 1, a positive high voltage is applied to the nozzle, so that the skin serves as a negative electrode. When an electric field is generated between the nozzle and the skin, the composition B at the nozzle tip portion is polarized by electrostatic induction to form the tip portion of the composition into a cone shape, and droplets of the charged composition B are discharged from the cone tip along the electric field into the air toward the skin. When the component (e) which is a solvent is evaporating from the composition B discharged into the space and charged, the charge density of the surface of the composition B becomes excessive, and the composition B spreads into the space while being repeatedly micronized by a coulomb repulsive force, and reaches the skin. Here, while the composition B is discharged into the space, the component (e) which is a volatile substance used as a solvent is volatilized from the composition, and the water-insoluble polymer capable of forming a coating film, which is a solute, is solidified, and simultaneously elongationally deformed by a potential difference to form fiber. The fiber can be deposited on the skin surface. In this way, a fiber assembly composed of a fiber deposit is formed on the skin surface. Adjustment of the distance between the nozzle and the skin or the voltage applied to the nozzle also enables the formation.

While the electrostatic spraying method is carried out, a high potential difference is generated between the nozzle and the skin which is a discharge target. However, the current passing through the human body is extremely small because the impedance is very large. For example, the present inventors have confirmed that a current passing through the human body while the electrostatic spraying method is carried out is a few orders of magnitude smaller than a current caused to pass through the human body by static electricity generated under everyday life.

The coating film for human skin in the present invention comprises the liquid preparation X on the surface of fibers or between fibers in the fiber assembly. The external composition A is applied to the skin before or after formation of the fiber to form the liquid preparation X at the application part of the fiber assembly, and the liquid preparation X comprises a liquid component which is applied to the skin together with the fiber during formation of the fiber assembly. The liquid preparation X is free of a volatile component such as an alcohol.

The composition of the liquid preparation X is that of the coating film on the skin, and since water or the like evaporates or adheres to the skin surface after elapse of time, the basis weight and the composition of the liquid preparation X are the basis weight and the composition immediately after formation of the coating film, and the values thereof are calculated from the applied composition A and composition B.

The basis weight P of the liquid preparation X in the present invention is from 0.5 to 2.3 mg/cm$^2$ from the viewpoint of use impressions for suppression of a feeling of pressure on the skin, transfer to clothes and stickiness, the viewpoint of formability of a mixed layer of the fiber deposit and the liquid preparation X and the viewpoint of vapor permeability of the coating film for human skin. From the same viewpoints, the basis weight of the liquid preparation X is preferably from 0.7 to 2.0 mg/cm$^2$, more preferably from 0.8 to 1.8 mg/cm$^2$. From the same viewpoints, the basis weight of the external composition A for forming the liquid preparation X, which is applied to the skin, is preferably from 0.5 to 2.3 mg/cm$^2$, more preferably from 0.7 to 2.0 mg/cm$^2$, further more preferably from 0.8 to 1.8 mg/cm$^2$.

The ratio of the basis weight P of the liquid preparation X or the external composition A in the present invention to the basis weight Q of the fiber deposit (P/Q) is 2.5 or more, preferably 3 or more, and 10 or less, preferably 8 or less, from the viewpoint of the balance between the fiber deposit and the liquid preparation X in the fiber layer for human skin in the present invention and the viewpoint of stably securing the vapor permeability of the coating film.

The relative permittivity of the liquid preparation X or the external composition A in the present invention is a total value obtained by multiplying a mass ratio by the relative permittivities of the oil agent in a liquid state at 20°C and the polyol in a liquid state at 20°C in the liquid preparation X or the external composition A. Here, the polyol in a liquid state at 20°C may be absent. In calculation of the mass ratio in the liquid preparation X or the external composition A, a mass ratio of each component to the mass of components excluding water is calculated for taking into account the relationship with vapor permeability. This is because the present inventors considered that the water evaporability is influenced by the relative permittivities of the liquid oil and the polyol except for those that are physically obstructive, such as solid fats. Therefore, the value of the water evaporability estimated from the determined relative permittivity, in other words, the vapor permeability of the coating film, is decreased (water evaporation is suppressed) by addition of a component which is physically obstructive, such as a solid fat. It is considered that when such a relative permittivity is below a certain value, it is possible to obtain a coating film which moderately suppresses water evaporation. The relative permittivity of the liquid preparation X and the external composition A in the present invention from the oil agent in a liquid state at 20°C and the polyol in a liquid state at 20°C is 2 or more and 27 or less, preferably 2 or more and 25 or less, more preferably 2 or more and 23 or less from the viewpoint of obtaining a coating film having moderate vaporability.

The permittivities of the oil agent in a liquid state at 20°C and the polyol in a liquid state at 20°C can be measured under conditions of a measurement terminal (SH-Z), 25°C, $\phi$10 mm and a distance of 1 mm using an impedance measuring device (SI1260 manufactured by Solartron).

The relative permittivity is a value obtained by dividing the permittivity of each component by the permittivity of vacuum. For example, the permittivity of vacuum is known to be 8.85 pF/m, and given that the permittivity of squalane oil is 7.19 pF/m, the relative permittivity of the squalane oil is 0.8.

The relative permittivity of the liquid preparation X or the external composition A in the present invention is a value obtained by multiplying the relative permittivities of components (a) and (b) as described later in the liquid preparation X or the external composition A by the mass ratios of the components (a) and (b), respectively, and total the mass ratios, and the aforementioned mass ratio is a mass ratio of each component to the mass of components in the liquid preparation X excluding volatile components such as water and ethanol.

In the present invention, the liquid preparation X or the external composition A comprises the component (a): oil agent in a liquid state at 20°C, preferably one or more selected from the group consisting of ester oil, hydrocarbon oil, silicone oil and higher alcohol which are liquid at 20°C. In the present invention, the oil agent in a liquid state at 20°C may have fluidity, and oil agents having no solid are defined as being liquid. For example, vaseline does not have high fluidity like that of water or olive oil, but has fluidity causing the form to change and is not solid at 20°C. Therefore, vaseline is considered liquid, and classified as the component (a) in the present invention.

The component (a): oil agent in a liquid state at 20°C in the liquid preparation X or the external composition A in the present invention has a relative permittivity of 0.5 or more and 20 or less, preferably 0.5 or more and 15 or less, from the viewpoint of controlling vapor permeability in combination with the fiber assembly.

The ester oil in the component (a) is oil in a liquid state at 20°C and has an ester structure with a HLB value of 8 or less. Here, the HLB value is an index of a hydrophile-lipophile balance, and in the present invention, a value calculated in accordance with the following equation by ODA and TERAMULA et al.

(Expression 1)

$$HLB = (\Sigma inorganic\ value / \Sigma organic\ value) \times 10$$

Examples of the ester oil in a liquid state at 20°C in the component (a) include esters composed of a linear or branched fatty acid and a linear or branched alcohol or polyhydric alcohol. Specific examples thereof include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di(2-ethylhexanoate), dipentaerythritol fatty acid esters, N-alkyl glycol monoisostearates, neopentyl glycol dicaprate, diisostearyl malate, glycerin di(2-heptylundecanoate), trimethylolpropane tri(2-ethylhaxanoate), trimethylolpropane triisostearate, pentaerythrite tetra(2-ethylhexanoate), glyceryl tri(2-ethylhexanoate), trimethylolpropane triisostearate, cetyl 2-ethylhexanoate, 2-ethylhexyl palmitate, diethylhexyl naphthalenedicarboxylate, alkyl benzoates (having 12 to 15 carbon atoms), cetearyl isononanoate, tri(caprylic acid/capric acid) glycerin, (dicaprylic acid/capric acid) butylene glycol, glyceryl trilaurate, glyceryl trimyristate, glyceryl tripalmitate, glyceryl triisostearate, glyceryl tri(2-heptylundecanoate), glyceryl tribehenate, glyceryl tricocoate, castor oil fatty acid methyl esters, oleyl oleate, 2-heptylundecyl palmitate, diisobutyl adipate, N-lauroyl-L-glutamic acid-2-octyldodecyl ester, di2-heptylundecyl adipate, ethyl laurate, di(2-ethylhexyl) sebacate, 2-hexyldecyl myristate, 2-hexyldecyl palmitate, 2-hexyldecyl adipate, diisopropyl sebacate, di(2-ethylhexyl) succinate, triethyl citrate, 2-ethylhexyl p-methoxy cinnamate and tripropylene glycol dipivalate.

Of these ester oils, at least one selected from the group consisting of octyldodecyl myristate, myristyl myristate, isocetyl stearate, isocetyl isostearate, cetearyl isononanoate, diisobutyl adipate, di2-ethylhexyl sebacate, isopropyl myristate, isopropyl palmitate, diisostearyl malate, neopentyl glycol dicaprate, alkyl benzoates (having 12 to 15 carbon atoms) and tri(caprylic acid/capric acid) glycerin is preferable, at least one selected from the group consisting of isopropyl myristate, isopropyl palmitate, diisostearyl malate, neopentyl glycol dicaprate, alkyl benzoates (having 12 to 15 carbon atoms) and tri(caprylic acid/capric acid) glycerin is more preferable, and at least one selected from the group consisting of neopentyl glycol dicaprate, alkyl benzoates (having 12 to 15 carbon atoms) and tri(caprylic acid/capric acid) glycerin is further more preferable, from the viewpoint of decreasing the permittivity to suppress the vapor permeability of the coating film (viewpoint of suppressing water evaporation) and the viewpoint of being excellent in use impression.

The triglyceride is preferably a fatty acid triglyceride, and is contained in, for example, olive oil, jojoba oil, macadamia nut oil, meadowfoam oil, castor oil, safflower oil, sunflower oil, avocado oil, canola oil, apricot kernel oil, rice germ oil, rice bran oil and the like.

Examples of the hydrocarbon oil in a liquid state at 20°C in the component (a) include liquid paraffin, squalane, squalene, n-octane, n-heptane, cyclohexane, light isoparaffin and liquid isoparaffin as well as pasty hydrocarbon oils such as vaseline, and from the viewpoint of improving the use impression, liquid paraffin, squalane and vaseline are preferable. From the viewpoint of suppressing the vapor permeability of the coating film of the present invention (suppressing water evaporation from the skin), the viscosity of the hydrocarbon oil at 30°C is preferably 10 mPa·s or more, more preferably 30 mPa·s or more. From such a viewpoint, the content of isododecane, isohexadecane or hydrogenated polyisobutene having a viscosity of less than 10 mPa·s at 30°C in the liquid preparation X or the external composition A is preferably 10 mass% or less, more preferably 5 mass% or less, further more preferably 1 mass% or less, even more preferably 0.5 mass% or less, and may be zero. The viscosity here is measured by a BM-type viscometer at 30°C (manufactured by Tokimec, Inc., measurement conditions: Rotor No. 1, 60 rpm, 1 minute). Since the component (a) is liquid at 20°C, the upper limit of the viscosity thereof at 30°C may be in a range allowing the component (a) to have fluidity, and is preferably 10,000 mPa·s or less, more preferably 2,000 mPa·s or less.

Examples of the silicone oil in the component (a) include dimethylpolysiloxane, dimethylcyclopolysiloxane, methylphenylpolysiloxane, methylhydrogenpolysiloxane and higher alcohol-modified organopolysiloxane, and of these, dimethylpolysiloxane is preferable from the viewpoint of a use impression.

Examples of the higher alcohol in the component (a) include liquid higher alcohols having 12 to 20 carbon atoms. Higher alcohols of branched fatty acids or unsaturated fatty acids are preferable, and examples thereof include decyltetradecanol, octyldodecanol, isostearyl alcohol, hexyldecanol and oleyl alcohol.

From the viewpoint of imparting moderate vapor permeability to the coating film while maintaining a good use impression, the content of the component (a) in the liquid preparation X or the external composition A in the present invention is preferably 100 mass% or less, and preferably 3 mass% or more, more preferably 5 mass% or more, as long as the relative permittivity range in the present invention is satisfied.

The liquid preparation X or the external composition A in the present invention may contain the component (b): polyol in a liquid state at 20°C. The content of the component (b) in the liquid preparation X is 50 mass% or less. From the viewpoint of suppressing the feeling of pressure on the skin while moderately suppressing the vapor permeability of the coating film for the skin of the present invention, the content of the component (b) in the liquid preparation X is more preferably 30 mass% or less, further more preferably 25 mass% or less, and preferably 2 mass% or more, more preferably 5 mass% or more when the component (b) is contained.

The liquid preparation X or the external composition A in the present invention may be free of the component (b), and when the component (b) is contained, the mass ratio of the component (b) to the component (a) (b/a) is preferably 5 or less, more preferably 4 or less, and preferably 0 or more, more preferably 1 or more, from the viewpoint of suppressing the feeling of pressure on the skin while moderately suppressing the vapor permeability of the coating film for skin of the present invention.

Examples of the polyol which is the component (b) include alkylene glycols such as ethylene glycol, propylene glycol, 1,3-propanediol and 1,3-butanediol; polyalkylene glycols such as diethylene glycol, dipropylene glycol, polyethylene glycol having a weight average molecular weight of 2,000 or less, and polypropylene glycol; and glycerins such as glycerin, diglycerin and triglycerin. Of these, from the viewpoint of the use impression such as smoothness during application, ethylene glycol, propylene glycol, 1,3-butanediol, dipropylene glycol, polyethylene glycol having weight average molecular weight of 2,000 or less, glycerin and diglycerin are preferable, and propylene glycol, 1,3-butanediol and glycerin are more preferable.

The liquid preparation X or the external composition A in the present invention may contain the component (c): solid fat which is solid at 20°C. The solid fat which is solid at 20°C has an effect of decreasing the vapor permeability of the coating film. From the viewpoint of suppressing the feeling of pressure on the skin by suppressing excessive obstruction resulting from an extreme decrease in vapor permeability of the coating film, when the solid fat which is solid at 20°C is contained, it is preferable that the component (b) be contained when the component (c): solid fat which is solid at 20°C is contained, and the mass ratio of the content of the component (c) and the component (b): polyol in a liquid state at 20°C (b/c) is preferably 1 or more, more preferably 1.5 or more, further more preferably 2 or more from the viewpoint of further improving a good use impression, and preferably 10 or less, more preferably 8 or less, further more preferably 6 or less.

While the liquid preparation X or the external composition A in the present invention may be free of the component (c), the content of the component (c) in the liquid preparation X or the external composition A is preferably 0 mass% or more. When the component (c) is contained, the content thereof is preferably 1 mass% or more, more preferably 2 mass% or more, further more preferably 3 mass% or more, and preferably 10 mass% or less, more preferably 8 mass% or less, from the viewpoint of securing a good use impression while limiting the vapor permeability of the coating film.

Examples of the component (c): solid fat which is solid at 20°C include cholesterol, higher alcohols of linear saturated fatty acids having 14 or more carbon atoms, fatty acid glyceryls of linear saturated fatty acids having 14 or more carbon atoms, linear saturated fatty acids having 14 or more carbon atoms, and ceramides. Examples of these solid fats include higher alcohols such as cetyl alcohol, stearyl alcohol and behenyl alcohol; fatty acid glyceryls such as glyceryl behenate; ceramides such as sphingosine, dihydrosphingosine and phytosphingosine; and fatty acids such as myristic acid, palmitic acid, stearic acid and behenic acid.

The external composition A and the liquid preparation X in the present invention may contain the component (d): water. Since the liquid preparation X is a coating film formed on the skin, and the component (d): water evaporates as time passes, the applied amount is taken as the amount of the component (d) in the liquid preparation X. The content of the component (d) in the liquid preparation X or the external composition A is preferably 0 mass% or more, more preferably 0.5 mass% or more, and preferably 80 mass% or less.

The external composition A in the present invention is in a liquid state, and includes emulsions, creams and pastes. The external composition A may be applied to the skin before or after formation of the fiber assembly. Examples of the method for the application include applying with hands or cotton, and spraying. Further, the coating film for human skin containing the liquid preparation X and the fiber assembly in the present invention may be applied to the skin for a certain time at a moderate vapor permeability. The application time is preferably 3 hours or more, more preferably 5 hours or more, and preferably 24 hours or less.

The vapor permeability of the coating film for human skin in the present invention is preferably from 300 to 3,000 $g/m^2 \cdot 24$ h, more preferably from 350 to 2,700 $g/m^2 \cdot 24$ h, further more preferably from 400 to 2,400 $g/m^2 \cdot 24$ h. Such a vapor permeability or maintenance of such a vapor permeability is difficult to achieve with conventional external compositions and cosmetics, and also difficult to achieve with a fiber deposit only, and a controlled vapor permeability can be more easily and sustainably achieved by the coating film of the present invention containing a deposit of specific fiber and the liquid preparation X.

In general, the healthy skin refers to a skin having an appearance without turn-up of horny cell layers and erythema and having high ability to retain water inside the body, and hence a moderately high moisture content, or a skin having a barrier function of preventing the inside of the body from external stimuli. Therefore, it is known that when the vapor permeability is high as in a fiber deposit or a cosmetic (water easily evaporates), the moisture content in the skin tends to decrease, and on the other hand, when the skin is sealed with, for example, a wrap film, the moisture content in the skin apparently increases, but the skin is swollen, so that the barrier function is rather deteriorated. In contrast, the coating film for human skin according to the present invention enables achievement of a moderate vapor permeability such that skin respiration is not hampered while suppressing water evaporation.

Now, it was found that application of coating film for human skin of the present invention having such vapor permeability improves skin metabolism. Here, the improvement of skin metabolism means enhancement of production of proteins necessary for formation of horny cell layers, and proteins necessary for increasing the moisture content in the skin, i.e., proteins involved in production of intercellular lipids and NMF. Examples thereof include enhancement of production of Suprabasin which is a marker of lamellar granules which are a source of ceramides and intercellular lipids, SASPase, filaggrin, bleomycin and caspase-14 which are involved in production of NMF, and transglutaminase which forms cellular skeletons. Enhancement of production of these proteins resultantly promotes improvement of skin metabolism to make the skin healthy, and consequently, it is possible to improve a damaged skin or improve skin tones. Accordingly, the coating film for human skin of the present invention can be used as a coating film for improving skin metabolism, a coating film for making the skin healthy (not claimed), a coating film for improving a damaged skin (not claimed), or a coating film for improving skin tones.

For the vapor permeability in the present invention, a coating film formed on the human skin is not directly measured, but a coating film produced under the same conditions is measured with the coating film considered to have an equivalent vapor permeability. The vapor permeability in the present invention is measured in accordance with the method of JIS Z2080 (1976), and for convenience, a mesh sheet (0.1 $\times$ 60 mesh, manufactured by Asa Dash Inc.) having an extremely high vapor permeability is used for retaining the fiber assembly and the liquid preparation X. The fiber assembly is formed on the mesh sheet by electrospinning, and the external composition A is applied to the fiber assembly, followed by performing the measurement. As a vapor-permeable cup, a screwing-type cup (manufactured by Imoto Machinery Co., Ltd.) specified in JIS Z2080 (1976) is used. Here, the coating film is formed in such a manner that the basis weight of the fiber assembly is 0.16 $mg/cm^2$, and the basis weight of the external composition A is 1 $mg/cm^2$. The time for spinning, the applied voltage, the discharge speed of the composition B from the device and the distance between the nozzle of the device and the mesh sheet are adjusted so that the fiber assembly has a fiber diameter of from 400 to 700 nm in an environment at 25°C and 30 RH.

The method for producing a coating film for human having an excellent vapor permeability as described above is preferably the following method. The method encompassed by the invention is defined in the appended claims.

A method for producing a coating film for human in which a coating film having a vapor permeability of from 300 to 3,000 $mg/cm^2 \cdot 24$ h is formed on a skin surface, the method including the steps of: applying an external composition A to the skin at a basis weight of from 0.5 to 2.3 $mg/cm^2$; and applying a composition B to the skin by an electrospinning method to form a fiber assembly having a basis weight of from 0.08 to 1.0 $mg/cm^2$, wherein the external composition A contains the following component (a) and has a relative permittivity of from 1.0 to 27, and the composition B contains 40 to 95 mass% of the following component (e) and 5 to 35 mass% of the following component (f) in the composition B:

component (a): oil agent in a liquid state at 20°C and which has a relative permittivity of 0.5 to 20;
component (e): one or more volatile substances selected from alcohols; and
component (f): water-insoluble polymer capable of forming a coating film.

Regarding the embodiments described above, disclosed are coating films, methods for producing a coating film, and use for improving skin metabolism, as shown hereafter.

Examples

Hereinafter, the present invention will be described in more detail by way of Examples. However, the scope of the present invention is not limited to such Examples. Examples of the present disclosure form part of the subject-matter for which protection is sought only if they fall within the scope of the claims.

Unless otherwise specified, "%" means "mass%".

[Example 1]

(1) Preparation of composition for fiber

Ethanol (manufactured by Wako Pure Chemical Industries, Ltd.; trade name: Ethanol (99.5%)) was used as a component (e) of the composition for fiber. Polyvinyl butyral as described later was used as a component (f). Ion-exchanged water was used as a component (g). Each component was measured and taken into a glass container to the amounts shown in Table 1, and was stirred with a magnetic stirrer at room temperature for about 12 hours to obtain a uniform and transparent solution. With this solution as a composition for fiber, a composition for electrospinning in this Example, a fiber deposit layer was formed by an electrospinning method under the conditions shown below.

[Table 1]

|  | Composition 1 for electrospinning | Composition 2 for electrospinning |
|---|---|---|
| Ethanol | 83.98 | 79.6 |
| Polyvinyl butyral (*1) | 11 | 12 |
| Di(phytosteryl-2-octyldodecyl) N-lauroyl-L-glutamate (*2) | 4.6 | - |
| Dimethylpolysiloxane (*3) | - | 4 |
| Polyethylene glycol (*4) | - | 4 |
| Water | 0.42 | 0.4 |
| Total | 100 | 100 |

(*1) S-LEC B BM-1 (SEKISUI CHEMICAL CO., LTD.)
(*2) Eldew PS203 (Ajinomoto Co., Inc.)
(*3) KF-96A-6CS (Shin-Etsu Chemical Co., Ltd.)
(*4) PEG400 (Sanyo Chemical Industries, Ltd.)

(2) Application of external composition A (1)

An area of 5 cm × 5 cm on the outer side of the shin of each of 17 subjects was set as a test site, and the skin external preparation 1 described in Table 2 was applied as an external composition A to the test site at 1.4 mg/cm². The subjects are women feeling a damaged skin and having a severe damaged skin (African-Americans or Caucasians from 35 to 55 years of age).
In group I, the skin external preparation 1 was applied to the outer side of the shin of one leg, followed by forming a fiber layer from a composition for electrospinning as described later (Example 1). In group II, only the skin external preparation 1 was applied to the outer side of the shin of the other leg, and a fiber layer was not provided (Comparative Example 1). In group III, the skin external preparation 1 was applied to the outer side of the shin, followed by attaching Tegaderm (1626W manufactured by 3M Company) which is an obstruction film (Comparative Example 4).

The skin external preparation 1 was used in this manner once daily for two weeks. Evaluation was performed once before the start of the use (base), one week after and two weeks after the start of the use, and one week after the end of the use (regression phase).

In group I (Example 1), the skin external preparation 1 was applied and a fiber layer was formed from the later-described composition for electrospinning once daily. On holidays, only the application of the skin external preparation 1 was performed.

[Table 2]

| | Skin external preparation 1 | (mass%) |
|---|---|---|
| (c) | Cetyl alcohol | 3.54 |
| | Cetyl-PG-hydroxyethyl | 0.50 |
| (a) | Isopropyl palmitate | 1.10 |
| | Vaseline | 3.75 |
| | Dimethylpolysiloxane (10cst) (*5) | 1.75 |
| (b) | Glycerin | 21.00 |
| | Behentrimonium chloride | 1.87 |
| | Methylparaben | 0.30 |
| | Purified water | 66.19 |
| | Total | 100.00 |
| | Relative permittivity | 14.6 |
| Q | Basis weight of fiber deposit (mg/cm$^2$) | 0.35 |
| | Basis weight of skin external preparation 1 (mg/cm$^2$) | 1.40 |
| P | Liquid preparation in fiber layer (mg/cm$^2$) | 1.55 |
| | P/Q | 4.0 |
| | Vapor permeability of coating film g/m$^2$·24h | 1489 |

(3) Formation of fiber assembly by electrospinning

By using an electrospinning device 10 having a configuration shown in Figure 2 and an appearance shown in Figure 1, a fiber assembly was formed on the area skin external preparation was applied under the following conditions from a composition for electrospinning 1 as described in Table 1.

- Applied voltage: 10 kV
- Distance between nozzle and skin: 100 mm
- Discharge time: 60 sec
- Discharge rate of composition for electrospinning: 0.10 mL/min
- Environment: 21°C ± 1°C, 40%RH

In this coating film by electrospinning, the basis weight of the (f) water-insoluble polymer in the fiber assembly is 0.35 mg/cm$^2$ as calculated on the premise that the specific gravity of the discharge liquid is 0.8. The fiber diameter is from 400 to 700 nm.

Evaluation on the test site was performed in the following manner. Before the evaluation, the outer side of the shin was washed with Cetaphil Daily Facial Wash, and water was removed, followed by conditioning for 15 minutes. Measurement for the evaluation was performed in an environment-variable room (room temperature: 21 ± 1°C and humidity: 40%RH).

[Evaluation 1] Skin roughness

A dedicated trained evaluator visually evaluated test sites by roughness scores on a scale of 0 to 4 (at intervals of 0.5). The score of a skin having no roughness is 0, and the score of a damaged skin is 4. Table 3 shows averaged results of evaluation on each subject.

As shown in Table 3, the coating film of the present invention in Example 1 exhibited significant improvement of skin roughness scores after one week and exhibited an improvement tendency after two weeks as compared to Comparative Example 1 in which only the skin external preparation 1 was applied.

[Table 3]

| Roughness score | Comparative Example 1 (only skin external preparation 1) | Example 1 (skin external preparation 1 + fiber assembly) |
|---|---|---|
| Base | 2.47±0.80 | 2.38±0.93 |
| One week | 1.91±1.06 | 1.29±0.95** |
| Two weeks | 0.74±0.77 | 0.38±0.49† |
| Regression | 1.38±0.96 | 1.24±0.79 |
| (average value ± standard deviation) (†: $p < 0.1$, **: $p < 0.01$: Wilcoxon signed-rank sum test) | | |

[Evaluation 2] Moisture content in horny cell layers (conductance)

The moisture content in horny cell layers was measured with Dermalab (manufactured by Cortex Technology, Ltd.). The moisture content in horny cell layers at the test site in each of Example 1 and Comparative Examples 1 and 4 was measured, and the average of measured values for each subject was determined. Tables 4 and 5 show the measurement results.

As shown in Tables 4 and 5, the coating film of the present invention containing the skin external preparation 1 and the fiber layer of Example 1 exhibited significant improvement of the moisture content in horny cell layers (conductance) after one week, after two weeks and at regression as compared to the Comparative Example 1 group in which only the skin external preparation 1 was applied.

The coating film of the present invention containing the skin external preparation 1 and the fiber layer of Example 1 exhibited significant improvement of the moisture content in horny cell layers (conductance) after one week and after two weeks as compared to the Comparative Example 4 group in which Tegaderm which is an obstruction film was attached after application of the skin external preparation 1.

[Table 4]

| ΔMoisture content in horny cell layer (conductance) | Comparative Example 1 | Example 1 |
|---|---|---|
| Base | 0.00±0.00 | 0.00±0.00 |
| One week | 28.94±12.47 | 41.65+13.02** |
| Two weeks | 35.06±12.39 | 49.65±14.06** |
| Regression | 9.18±9.70 | 15.29±10.44* |
| (average value ± standard deviation) (*: $p < 0.05$, **: $p < 0.01$: paired t-test, two sided) | | |

[Table 5]

| ΔMoisture content in horny cell layer (conductance) | Comparative Example 4 | Example 1 |
|---|---|---|
| Base | 0.00±0.00 | 0.00±0.00 |
| One week | 33.76±16.46 | 41.65±13.02## |
| Two weeks | 36.35±13.84 | 49.65±14.06## |
| Regression | 10.24±8.39 | 15.29±10.44 |
| (average value ± standard deviation) (##: $p < 0.01$: Bonferroni test) | | |

[Evaluation 3] Analysis of proteins in horny cell layers

A tape (obtained by cutting Film Masking Tape 465#40 from TERAOKA SEISAKUSHO CO., LTD. into a size of 2.5 cm × 5.0 cm) was pressed against a skin surface, and a total of six sheets were collected from three different regions with two sheets in sequence collected from each of the regions.

The collected tapes were immersed in 1 mL of an aqueous solution (aqueous solution of a mixture of urea at 7 mol/L, thiourea at 2 mol/L, sodium deoxycholate at 12 mmol/L, SLS water at 12 mmol/L and Tris-HCl (pH 9) at 100 mmol/L), and subjected to ultrasonic treatment in iced water for 20 minutes. 10 μL of an aqueous solution of a mixture of dithiothreitol at 1 mol/L and ammonium bicarbonate at 50 mmol/L was added thereto, and the mixture was shaken at 37°C overnight. 50 μL of a mixed aqueous solution of iodo acetoamide at 1 mol/L and ammonium bicarbonate at 50 mmol/L was added thereto, and the mixture was left to stand at room temperature for 30 minutes to give a protein extraction solution.

This was left to stand at room temperature for 30 minutes to give a protein extraction solution.

The amount of protein was determined by the EZQ protein assay.

2 mL of an aqueous ammonium bicarbonate solution at 50 mmol/L was added to the extraction solution, a Lys-C solution prepared at 0.2 μg/μL was added at one-hundredth the weight of protein, and the mixture was shaken at 37°C for 3 hours. Thereafter, a trypsin solution prepared at 0.2 μg/μL was added at one-hundredth the amount of protein, and the mixture was shaken at 37°C for 20 hours. The sample solution was divided among 2 mL tubes, and to each of the tubes, 1 mL of ethyl acetate and a 50% aqueous TFA solution were each added at a final concentration of 0.5%. Thereafter, the mixture was stirred for 2 minutes, and centrifuged at 15,000 r/min for 3 minutes. The ethyl acetate in the upper layer was removed, and the remaining sample solution was dried under reduced pressure at 50°C. Thereafter, the sample was demineralized, and dissolved by addition of a 0.1% TFA-containing 2% aqueous acetonitrile solution to prepare a sample solution at a final protein concentration of 1 μg/μL.

(LC-MS measurement)

For the prepared sample, LC-MS measurement was performed in the following manner.

NanoAcquity UPLC System with 2D Technology (trade name) (manufactured by Waters Corporation) was used as liquid chromatography (hereinafter, also referred to as "LC"), and LTQ Ortbitrap Velos (trade name) (manufactured by Thermo Fisher Scientific) as a mass spectrometer (hereinafter, also referred to as "MS").

LC conditions and MS conditions are as shown below.

(1) LC conditions

Device: NanoAcquity UPLC (manufactured by Waters Corporation)
Trap column: Nano Ease Xbridge BEH130 C18 (trade name), particle size: 5 μm, 0.3 mm (inner diameter) × 50 mm (length) (manufactured by Waters Corporation)
Sample loading solution: 0.1% aqueous formic acid solution : 0.1% formic acid-containing acetonitrile = 98 : 2
Washing at 5 μL/min for 3 minutes after injection of sample
Separation column: NanoAcquity UPLC BEH130 C18 (trade name), particle size: 1.7 μm, 0.1 mm (inner diameter) × 100 mm (length) (manufactured by Waters Corporation) Eluent A: 0.1% aqueous formic acid solution
Eluent B: 0.1% formic acid-containing acetonitrile
Flow rate: 400 nL/min
Gradient: eluent B 5% (from 0 to 1 minute) → 40% (120 minutes) → 95% (from 121 to 130 minutes) → 5% (from 131 to 140 minutes)
Injection amount: 1 μL
Column temperature: 35°C

(2) MS conditions

Ionization method: nano-electrospray ionization (nano ESI) method
Spray tip: Pico Tip NanoSpray Emitter FS360-50-15-N (trade name), outer diameter: 360 μm, tip outer diameter: 50 μm, inner diameter: 15 μm (New Objective)
Polarity: positive
Spray voltage: 1,800 V

Capillary temperature: 250°C
Range (FT-MS): m/z from 300 to 1,250
Mass resolution: 60,000 (at m/z 400)
MS/MS (IT-MS): collision-induced dissociation (CID) method
Data dependent scan: Top15
Exclusion time: 180 s
Range (IT-MS): m/z from 100 to 2,000
Collision Energy (CE): 35

For identification of enzymatic digests of each protein, Swiss-Prot (http://web.expasy.org/docs/swiss-prot_guide line.html) was used as a database, MASCOT Server (Matrix Science) was used as a database search engine, and Proteome Discoverer (Themo Fisher Scientific) was used as analysis software.

Search conditions are as shown below.

(3) Protein database search

Analysis software: Proteome Discoverer ver. 1.3
Search engine: Mascot ver. 2.3
Database: Swiss-Prot 2012/02
Enzyme: Trypsin
Static modification: carbamidomethyl (C)
Variable modification: oxidation (M)
Maximum missed cleavage: 1
Precursor Mass tolerance: $\pm 10$ ppm
Fragment Mass tolerance: $\pm 0.8$ Da

(4) Protein identification conditions

FDR: 5%
Peptide Rank: 1

With respect to the acquired LC-MS data, the file format was converted from the thermo Fisher Scientific's unique '. raw' format to '. mzML', which is a comprehensive format. For the conversion of data format, msconvert from Proteo Wizard was used to convert into . mzML file which is a general data interchange format.

The data converted into the . mzML format was analyzed by using R ver. 3.0.1 (http://www.r-project.org/). For the analysis, packages delivered from CRAN (The Comprehensive R Archive Network, http://cran.r-project.org/) and Bioconductor (http://www.bioconductor.org/) were used. (mzR, doParallel, compiler, Matrix).

Details of the analysis method are as shown below.

Extraction ion chromatographs for the m/z of all peptides used for assignment of the proteins were obtained. When a target peptide was found, a peak area of the extraction ion chromatogram was calculated. When a target peptide was not found, the retention time was corrected on the basis of a peptide detected in all samples, and the signal intensity in a retention time range at which the peptide was supposed to be present was defined as a peak area. Then, a value obtained by averaging the peak areas of relevant peptides for each protein was treated as the amount of protein.

Table 6 shows the amount of protein after two weeks as a ratio of the amount of protein in Example 1 (skin external preparation 1 and fiber layer) to that in Comparative Example 1 (only skin external preparation 1).

As shown in Table 6, production of proteins necessary for formation of horny cell layers, and proteins essential for increasing the moisture content in the skin, i.e., proteins involved in production of intercellular lipids and NMF, was enhanced in Example 1 as compared to Comparative Example 1. Specifically, production of Suprabasin which is a marker of lamellar granules which are a source of ceramides and intercellular lipids, SASPase, filaggrin, bleomycin and caspase-14 which are involved in production of NMF, and transglutaminase which forms cellular skeletons was enhanced.

[Table 6]

| Protein type | Description of protein | Amount of protein produced after two weeks (Example 1/Comparative Example 1) |
|---|---|---|
| A2ML1 | Desquamation | 2.7 |
| Suprabasin | marker of Lamellar bodies | 2.2 |
| GGCT | Production of NMF | 2.1 |
| Calpain-1 subunit | Production of NMF | 2.0 |
| Filaggrin | Production of NMF | 1.9 |
| TGM1 | Cornification | 1.7 |
| Filaggrin-2 | Production of NMF | 1.7 |
| KLK7 | Desquamation | 1.3 |
| TGM3 | Cornification | 1.5 |
| Arginase-1 | Production of NMF | 1.5 |
| Caspase-14 | Production of NMF | 1.4 |
| Bleomycin | Production of NMF | 1.3 |
| SASPase | Production of NMF | 1.3 |

**A2ML1 :alpha-2-macroglobulin-like-1**
**GGCT: gamma-glutamyl cyclotransferase**
**TGM1: Transglutaminase 1**
**KLK7: kallikrein 7**
**TGM3: Transglutaminase 3**

[Evaluation 4] Vapor permeability

The vapor permeability of each of the coating film of the skin external preparation 1 in Table 2 and the fiber assembly from the composition for electrospinning 2 in Table 1, the coating film of the skin external preparation 2 and the fiber assembly from the composition for electrospinning 2 in Table 1, and the coating film of the skin external preparation 3 and the fiber assembly from the composition for electrospinning 2 in Table 1 was measured. The vapor permeability is measured in accordance with the method of JIS Z2080 (1976). A mesh sheet (0.1 × 60 mesh, manufactured by Asa Dash Inc.) was used for retaining the fiber assembly and the skin external preparation 1, 2 or 3. The composition for electrospinning 2 was applied to the mesh sheet, and the fiber assembly was formed by the electrospinning method under the following conditions.

- Applied voltage: 10 kV
- Distance between nozzle and skin: 80 mm
- Discharge time: 30 sec
- Discharge area: 6 cm diameter
- Discharge rate of composition for electrospinning: 0.10 mL/min
- Environment: 21°C ± 1°C, 40%RH

In this coating film by electrospinning, the basis weight of the polymer in the fiber is 0.16 mg/cm$^2$ as calculated on the premise that the specific gravity of the discharge liquid is 0.8. The fiber diameter is from 400 to 700 nm.

Further, the skin external preparation 1, 2 or 3 was applied to prepare a coating film for measurement of the vapor permeability. Next, the vapor permeability was measured. As a vapor-permeable cup, a screwing-type cup (Imoto Machinery Co., Ltd.) specified in JIS Z2080 (1976) was used. The vapor permeability was measured by the method specified in JIS Z2080 (1976). Tables 2, 7 and 10 show the measurement results.

[Evaluation 5] Permittivity

The permittivities of the skin external preparation 1 shown in Table 2, the skin external preparation 2 shown in Table 7, the polyol and the oil which are liquid at 20°C in each of the oil agents 1 to 5 in Table 9, and the skin external preparation 3 shown in Table 10 are measured. The permittivity is measured under conditions of a measurement terminal (SH-Z), 25°C, $\phi$ 10 mm and a distance of 1 mm using an impedance measuring device (SI1260 manufactured by Solartron). A value obtained by dividing the measured permittivity by the permittivity of vacuum (8.85 pF/m) is defined as a relative permittivity. Further, the sum of products of the relative permittivity and the mass ratio to components contained in each of the skin external preparations 1, 2 and 3 and the oil agents 1 to 5, other than water, was calculated, and defined as the relative permittivity of each external composition. Tables 2, 7, 9 and 10 show the results.

(Example 2 and Comparative Example 2)

(1) The skin external preparation 2 shown in Table 7 was applied at a basis weight of 1 mg/cm$^2$ to an area of 5 × 7 cm of each of the left and right cheeks of 30 subjects. 45 women in their thirties to forties, who were highly concerned about dry skin or dryness and had a healthy skin, were employed as subjects.

(2) Formation of fiber assembly by electrospinning

Using an electrospinning device 10 having a configuration shown in Figure 1 and an appearance shown in Figure 2, a fiber assembly was formed on the area to which skin external preparation 2 was applied (5 × 7 cm) from the composition for electrospinning 2 described in Table 1 under the following conditions (N = 30), and a test was conducted as Example 2. A test on a group without formation of the fiber assembly (N = 15) was conducted as Comparative Example 2.

- Applied voltage: 10 kV
- Distance between nozzle and skin: 80 mm
- Discharge time: 35 sec
- Discharge rate of composition for spraying: 0.10 mL/min
- Environment: 21°C ± 1°C, 40%RH

In this coating film by electrospinning, the basis weight of the polymer in the fiber is 0.17 mg/cm$^2$ as calculated on the premise that the specific gravity of the discharge liquid is 0.8.

[Evaluation 2]

For Example 2 and Comparative Example 2, the moisture content in the horny cell layers was measured after application for 6 hours. The moisture content in the horny cell layers was measured using Corneometer CM825 (Courage + Khazaka Electronic GmbH). For Example 2 and Comparative Example 2, the moisture contents in the horny cell layers before and after the application were measured on the applied sites, and the difference between the moisture contents before and after the application was calculated. The average thereof was defined as measurement results. Table 8 shows the results. As shown in Table 8, the moisture content inthe skin was higher in Example 2 than Comparative Example 2.

[Table 7]

|  | Skin external preparation 2 | (mass%) |
|---|---|---|
| (c) | Ceramide | 1.7 |
|  | Cetyl alcohol | 0.8 |
|  | Glyceryl behenate | 1 |
|  | N-stearoyl-L-glutamic acid (*10) | 0.54 |
| (a) | Neopentyl glycol dicaprate | 3 |
|  | Diglyceryl diisostearate | 0.1 |
|  | Olive oil | 0.8 |
|  | Dimethylpolysiloxane 10CS (*5) | 4 |
|  | Dimethylpolysiloxane 50CS (*6) | 1 |
|  | Methylpolysiloxane/crosslinked methylpolysiloxane mixture (*7) | 0.36 |

(continued)

| | | Skin external preparation 2 | (mass%) |
|---|---|---|---|
| (b) | | Glycerin | 7 |
| | | 1,3-butylene glycol | 5.5 |
| | | Polyethylene glycol (*8) | 1.5 |
| | | Carbopol (*9) | 0.14 |
| | | L-arginine | 0.255 |
| | | Potassium hydroxide | 0.09 |
| | | Methyl paraoxybenzoate | 0.35 |
| | | Ethyl paraoxybenzoate | 0.05 |
| | | Purified water | 71.815 |
| | | Relative permittivity | 18.3 |
| Q | | Basis weight of fiber deposit (mg/cm$^2$) | 0.17 |
| | | Basis weight of applied skin external preparation 2 (mg/cm$^2$) | 1.00 |
| P | | Basis weight of liquid preparation in fiber layer (mg/cm$^2$) | 1.11 |
| | | P/Q | 6.5 |
| | | Vapor permeability of coating film g/m$^2$·24h | 1776 |

(*5) KF-96A-10CS (Shin-Etsu Chemical Co., Ltd.)
(*6) KF-96A-50CS (Shin-Etsu Chemical Co., Ltd.)
(*7) KSG-16 (Shin-Etsu Chemical Co., Ltd.)
(*8) PEG-1540(-G) (Sanyo Chemical Industries, Ltd.)
(*9) ETD2020 POLYMER (US) (Belgium)
(*10) AMISOFT HA-P (Ajinomoto Co., Inc.)

[Table 8]

| | Comparative Example 2 (only skin external preparation 2) | Example 2 (skin external preparation 2 + fiber assembly) |
|---|---|---|
| ΔMoisture content in horny cell layer | 7.4 | 8.6 |

(Examples 3 to 6 and Comparative Example 3)

The coating films of Comparative Example 3 and Examples 3 to 6 were produced from the fiber assembly from the composition for electrospinning 1 in Table 1 and the oil agents 1 to 5 in Table 9, and the vapor permeability of each of the coating films was measured. The vapor permeability is measured in accordance with the method of JIS Z20080 (1976). A mesh sheet (0.1 × 60 mesh, manufactured by Asa Dash Inc.) was used for retaining the fiber assembly and the skin external preparation 1 or 2. The composition for electrospinning 1 was applied to the mesh sheet, and the fiber assembly was formed by the electrospinning method under the following conditions.

- Applied voltage: 10 kV
- Distance between nozzle and skin: 80 mm
- Discharge time: 30 sec
- Discharge area: 6 cm diameter
- Discharge rate of composition for electrospinning: 0.10 mL/min
- Environment: 21°C ± 1°C, 40%RH

In this coating film by electrospinning, the basis weight of the polymer in the fiber is 0.16 mg/cm$^2$ as calculated on the premise that the specific gravity of the discharge liquid is 0.8. The fiber diameter is from 400 to 700 nm.
The oil agents 1 to 5 were applied to prepare coating films of Comparative Example 3 and Examples 3 to 6 for measurement of the vapor permeability. Next, the vapor permeability was measured. As a vapor-permeable cup, a screwing-type cup (Imoto Machinery Co., Ltd.) specified in JIS Z2080 (1976) was used. The vapor permeability was measured by the method specified in JIS Z2080 (1976). Table 9 shows the measurement results. As shown in Table 9, the vapor permeability of the coating film increased as the value of the relative permittivity of the external composition became larger.

[Table 9]

|  | Comparative Example 3 | Example 3 | Example 4 | Example 5 | Example 6 |
|---|---|---|---|---|---|
| Oil agent (mass%) | Oil agent 1 | Oil agent 2 | Oil agent 3 | Oil gent 4 | Oil agent 5 |
| Squalane oil | 100 | 75 | 50 | 25 | 0 |
| Octyl paramethoxycinnamate (*11) | 0 | 25 | 50 | 75 | 100 |
| Total | 100 | 100 | 100 | 100 | 100 |
| Relative permittivity | 0.8 | 2 | 3.2 | 4.4 | 5.6 |
| Vapor permeability (g/m$^2$·24h) | 182 | 371 | 602 | 745 | 1077 |
| (*11) Uvinul MC80 (manufactured by BASF SE) | | | | | |

[Table 10]

| | Skin external preparation 3 | (mass%) |
|---|---|---|
| | Ceramide | 1 |
| (c) | Cetyl alcohol | 0.25 |
| | Glyceryl behenate | 0.5 |
| | N-stearoyl-L-glutamic acid (*10) | 0.27 |
| | Neopentyl glycol dicaprate | 3 |
| | Diglyceryl diisostearate | 0.1 |
| | Olive oil | 0.8 |
| (a) | Dimethylpolysiloxane 10CS (*5) | 4 |
| | Dimethylpolysiloxane 50CS (*6) | 1 |
| | Methylpolysiloxane/crosslinked methylpolysiloxane mixture (*7) | 0.36 |
| | Glycerin | 7 |
| (b) | 1,3-butylene glycol | 5.5 |
| | Polyethylene glycol (*8) | 1.5 |
| | Carbopol (*9) | 0.14 |
| | L-arginine | 0.189 |
| | Potassium hydroxide | 0.096 |
| | Methyl paraoxybenzoate | 0.35 |
| | Ethyl paraoxybenzoate | 0.05 |
| | Purified water | 74.895 |
| | Relative permittivity | 21.3 |
| | Vapor permeability of coating film g/m$^2$·24h | 2456 |
| (*5) KF-96A-10CS (Shin-Etsu Chemical Co., Ltd.)<br>(*6) KF-96A-50CS (Shin-Etsu Chemical Co., Ltd.)<br>(*7) KSG-16 (Shin-Etsu Chemical Co., Ltd.)<br>(*8) PEG-1540(-G) (Sanyo Chemical Industries, Ltd.)<br>(*9) ETD2020 POLYMER (US)(Belgium)<br>(*10) AMISOFT HA-P (Ajinomoto Co., Inc.) | | |

**Claims**

1.  A coating film for human skin comprising a fiber layer,

    wherein the fiber layer comprises: a fiber assembly in which fiber of a water-insoluble polymer with a fiber diameter of 50 nm or more and less than 10,000 nm is deposited; and a liquid preparation X present between fibers and on a surface of fibers in the fiber assembly,
    the fiber assembly has a basis weight Q of 0.08 mg/cm$^2$ or more and 1.0 mg/cm$^2$ or less, and
    the liquid preparation X has a basis weight P of 0.5 mg/cm$^2$ or more and 2.3 mg/cm$^2$ or less and a relative permittivity of 2 or more and 27 or less, and comprises a component (a): oil agent in a liquid state at 20°C and has a relative permittivity of 0.5 or more and 20 or less, and optionally a component (b): polyol in a liquid state at 20°C, in which the content of the component (b) in the liquid preparation X is 50 mass% or less;
    wherein the ratio of the basis weight P of the liquid preparation X to the basis weight Q of the fiber assembly (P/Q) is 2.5 or more and 10 or less:

    wherein the relative permittivity of the liquid preparation X is a total value obtained by multiplying a mass ratio by the relative permittivities of the oil agent in a liquid state at 20°C and the polyol in a liquid state at 20°C in the liquid preparation, wherein for calculation of the mass ratio in the liquid preparation X, a mass ratio of each component to the mass of components in the liquid preparation X excluding volatile components is calculated;
    wherein the volatile components have a vapor pressure at 20°C of 1.33 kPa or more and 40.00 kPa or less, and wherein the relative permittivity is determined as described in the section "[Evaluation 5] Permittivity" of the specification.

2.  The coating film for human skin according to claim 1, wherein the fiber assembly is formed directly on the human skin, and is formed by applying an external composition A comprising the component (a) before or after the formation of the fiber assembly.

3.  The coating film for human skin according to claim 2, wherein the fiber assembly is formed on the human skin by an electrospinning method.

4.  The coating film for human skin according to any one of claims 1 to 3, wherein the liquid preparation X comprises a component (c): oil which is solid at 20°C, and the mass ratio of the component (b) to the component (c) (b/c) is 8 or less.

5.  The coating film for human skin according to claim 2 or 3, wherein the external composition A comprises the component (c): oil which is solid at 20°C, and the mass ratio of the component (b) to the component (c), (b)/(c), in the external composition A is 8 or less.

6.  The coating film for human skin according to any one of claims 1 to 5, wherein the vapor permeability as measured in accordance with the method of JIS Z2080 (1976) is from 300 to 3,000 g/m$^2$·24 h.

7.  The coating film for human skin according to any one of claims 1 to 6, which is applied for 5 hours or more, at a vapor permeability of from 300 to 3,000 g/m$^2$·24 h.

8.  A non-therapeutic method for producing a coating film for human in which a coating film having a vapor permeability of from 300 to 3,000 g/m$^2$·24 h is formed on a skin surface,
    the method comprising the steps of:

    applying an external composition A to the skin at a basis weight of from 0.5 to 2.3 mg/cm$^2$; and
    applying a composition B to the skin by an electrospinning method to form a fiber assembly having a basis weight of from 0.08 to 1.0 mg/cm$^2$,
    wherein the external composition A comprises the following component (a) and may optionally comprise the following component (b) and has a relative permittivity of from 2 to 27, and
    the composition B comprises from 40 to 95 mass% of the following component (e) and from 5 to 35 mass% of the following component (f) in the composition B:

    component (a): oil agent in a liquid state at 20°C and which has a relative permittivity of from 0.5 to 20;
    component (b): a polyol in a liquid state at 20°C;

**EP 3 888 753 B1**

component (e): one or more volatile substances selected from the group consisting of alcohols; and
component (f): water-insoluble polymer capable of forming a coating film,
wherein the ratio of the basis weight P of the liquid preparation A to the basis weight Q of the fiber assembly
(P/Q) is 2.5 or more and 10 or less:

wherein the relative permittivity of the liquid preparation A is a total value obtained by multiplying a mass
ratio by the relative permittivities of the oil agent in a liquid state at 20°C and the polyol in a liquid state at
20°C in the liquid preparation, wherein for calculation of the mass ratio in the liquid preparation A, a mass
ratio of each component to the mass of components in the liquid preparation A excluding volatile
components is calculated;
wherein volatile components have a vapor pressure at 20°C of 1.33 kPa or more and 40.00 kPa or less,
and wherein the relative permittivity is determined as described in the section "[Evaluation 5] Permittivity"
of the specification.

**Patentansprüche**

1. Beschichtungsfilm für menschliche Haut, umfassend eine Faserschicht,

   wobei die Faserschicht umfasst: eine Faseranordnung, in der Fasern eines wasserunlöslichen Polymers mit
   einem Faserdurchmesser von 50 nm oder mehr und weniger als 10.000 nm angeordnet sind; und eine flüssige
   Zubereitung X, die zwischen den Fasern und auf einer Oberfläche der Fasern in der Faseranordnung vorliegt,
   wobei die Faseranordnung ein Flächengewicht Q von 0,08 mg/cm$^2$ oder mehr und 1,0 mg/cm$^2$ oder weniger
   aufweist, und
   die flüssige Zubereitung X ein Flächengewicht P von 0,5 mg/cm$^2$ oder mehr und 2,3 mg/cm$^2$ oder weniger und
   eine relative Permittivität von 2 oder mehr und 27 oder weniger aufweist und eine Komponente (a): Ölmittel im
   flüssigen Zustand bei 20°C und mit einer relativen Permittivität von 0,5 oder mehr und 20 oder weniger, und
   optional eine Komponente (b): Polyol im flüssigen Zustand bei 20°C, wobei der Gehalt der Komponente (b) in der
   flüssigen Zubereitung X 50 Massen-% oder weniger beträgt, umfasst;
   wobei das Verhältnis von dem Flächengewicht P der flüssigen Zubereitung X zu dem Flächengewicht Q der
   Faseranordnung (P/Q) 2,5 oder mehr und 10 oder weniger beträgt:

   wobei die relative Permittivität der flüssigen Zubereitung X ein Gesamtwert ist, der durch Multiplikation eines
   Massenverhältnisses mit den relativen Permittivitäten des Ölmittels im flüssigen Zustand bei 20°C und des
   Polyols im flüssigen Zustand bei 20°C in der flüssigen Zubereitung erhalten wird, wobei zur Berechnung des
   Massenverhältnisses in der flüssigen Zubereitung X ein Massenverhältnis jeder Komponente zu der Masse
   der Komponenten in der flüssigen Zubereitung X ohne flüchtige Komponenten berechnet wird;
   wobei die flüchtigen Komponenten einen Dampfdruck bei 20°C von 1,33 kPa oder mehr und 40,00 kPa oder
   weniger aufweisen und wobei die relative Permittivität, wie im Abschnitt "[Evaluation 5] Permittivity" der
   Spezifikation beschrieben, bestimmt wird.

2. Beschichtungsfilm für menschliche Haut gemäß Anspruch 1, wobei die Faseranordnung direkt auf der menschlichen
   Haut gebildet wird und durch Aufbringen einer externen Zusammensetzung A, umfassend die Komponente (a), vor
   oder nach dem Bilden der Faseranordnung gebildet wird.

3. Beschichtungsfilm für menschliche Haut gemäß Anspruch 2, wobei die Faseranordnung auf der menschlichen Haut
   durch ein Elektrospinnverfahren gebildet wird.

4. Beschichtungsfilm für menschliche Haut gemäß einem der Ansprüche 1 bis 3, wobei die flüssige Zubereitung X eine
   Komponente (c) umfasst: Öl, das bei 20°C fest ist, und das Massenverhältnis von der Komponente (b) zu der
   Komponente (c) (b/c) 8 oder weniger beträgt.

5. Beschichtungsfilm für menschliche Haut gemäß Anspruch 2 oder 3, wobei die externe Zusammensetzung A die
   Komponente (c) umfasst: Öl, das bei 20°C fest ist, und das Massenverhältnis von der Komponente (b) zu der
   Komponente (c), (b)/(c), in der externen Zusammensetzung A 8 oder weniger beträgt.

6. Beschichtungsfilm für menschliche Haut gemäß einem der Ansprüche 1 bis 5, wobei die Dampfdurchlässigkeit,
   gemessen gemäß dem Verfahren von JIS Z2080 (1976), 300 bis 3.000 g/m$^2$·24 h beträgt.

7. Beschichtungsfilm für menschliche Haut gemäß einem der Ansprüche 1 bis 6, der für 5 Stunden oder länger bei einer Dampfdurchlässigkeit von 300 bis 3.000 g/m²·24 h aufgetragen wird.

8. Nicht-therapeutisches Verfahren zur Herstellung eines Beschichtungsfilms für Menschen, bei dem ein Beschichtungsfilm mit einer Dampfdurchlässigkeit von 300 bis 3.000 g/m²·24 h auf einer Hautoberfläche gebildet wird, wobei das Verfahren die Schritte umfasst:

das Auftragen einer externen Zusammensetzung A auf die Haut mit einem Flächengewicht von 0,5 bis 2,3 mg/cm²; und

das Aufbringen einer Zusammensetzung B auf die Haut durch ein Elektrospinnverfahren, um eine Faseranordnung mit einem Flächengewicht von 0,08 bis 1,0 mg/cm² zu bilden,
wobei die externe Zusammensetzung A die folgende Komponente (a) umfasst und optional die folgende Komponente (b) umfassen kann und eine relative Permittivität von 2 bis 27 aufweist, und
die Zusammensetzung B 40 bis 95 Massen-% der folgenden Komponente (e) und 5 bis 35 Massen-% der folgenden Komponente (f) in der Zusammensetzung B umfasst:

Komponente (a): Ölmittel im flüssigen Zustand bei 20°C und mit einer relativen Permittivität von 0,5 bis 20;
Komponente (b): ein Polyol im flüssigen Zustand bei 20°C;
Komponente (e): eine oder mehrere flüchtige Substanzen, ausgewählt aus der Gruppe, bestehend aus Alkoholen; und
Komponente (f): wasserunlösliches Polymer, das einen Beschichtungsfilm bilden kann,
wobei das Verhältnis von dem Flächengewicht P der flüssigen Zubereitung A zu dem Flächengewicht Q der Faseranordnung (P/Q) 2,5 oder mehr und 10 oder weniger beträgt:

wobei die relative Permittivität der flüssigen Zubereitung A ein Gesamtwert ist, der durch Multiplikation eines Massenverhältnisses mit den relativen Permittivitäten des Ölmittels im flüssigen Zustand bei 20°C und des Polyols im flüssigen Zustand bei 20°C in der flüssigen Zubereitung erhalten wird,
wobei zur Berechnung des Massenverhältnisses in der flüssigen Zubereitung A ein Massenverhältnis jeder Komponente zu der Masse der Komponenten in der flüssigen Zubereitung A ohne flüchtige Komponenten berechnet wird;
wobei flüchtige Komponenten einen Dampfdruck bei 20°C von 1,33 kPa oder mehr und 40,00 kPa oder weniger aufweisen und wobei die relative Permittivität, wie im Abschnitt "[Evaluation 5] Permittivity" der Spezifikation beschrieben, bestimmt wird.

**Revendications**

1. Film de revêtement pour la peau humaine comprenant une couche fibreuse,

dans lequel la couche fibreuse comprend : un assemblage de fibres dans lequel est déposée une fibre d'un polymère insoluble dans l'eau, d'un diamètre compris entre 50 nm ou plus et moins de 10 000 nm ; et une préparation liquide X présente entre des fibres et sur une surface de fibres dans l'assemblage de fibres,
l'assemblage de fibres présente un poids de base Q compris entre 0,08 mg/cm² ou plus et 1,0 mg/cm² ou moins, et
la préparation liquide X présente un poids de base P compris entre 0,5 mg/cm² ou plus et 2,3 mg/cm² ou moins et une permittivité relative comprise entre 2 ou plus et 27 ou moins, et comprend un composant (a) : agent huileux dans un état liquide à 20 °C et présentant une permittivité relative comprise entre 0,5 ou plus et 20 ou moins, et éventuellement un composant (b) : polyol dans un état liquide à 20 °C, dans lequel la teneur en composant (b) dans la préparation liquide X est de 50 % en masse ou moins ;
dans lequel le rapport entre le poids de base P de la préparation liquide X et le poids de base Q de l'assemblage de fibres (P/Q) est compris entre 2,5 ou plus et 10 ou moins ;
dans lequel la permittivité relative de la préparation liquide X est une valeur totale obtenue en multipliant un rapport massique par les permittivités relatives de l'agent huileux dans un état liquide à 20 °C et du polyol dans un état liquide à 20 °C dans la préparation liquide, dans lequel, pour le calcul du rapport massique dans la préparation liquide X, un rapport massique de chaque composant par rapport à la masse de composants dans la préparation liquide X, à l'exclusion de composants volatils, est calculé ;
dans lequel les composants volatils présentent une pression de vapeur à 20 °C comprise entre 1,33 kPa ou plus et 40,00 kPa ou moins,

et dans lequel la permittivité relative est déterminée comme décrit dans la section « [Évaluation 5] Permittivité » de la spécification.

2. Film de revêtement pour la peau humaine selon la revendication 1, dans lequel l'assemblage de fibres est formé directement sur la peau humaine, et est formé en appliquant une composition externe A comprenant le composant (a) avant ou après la formation de l'assemblage de fibres.

3. Film de revêtement pour la peau humaine selon la revendication 2, dans lequel l'assemblage de fibres est formé sur la peau humaine par un procédé d'électrofilage.

4. Film de revêtement pour la peau humaine selon l'une quelconque des revendications 1 à 3, dans lequel la préparation liquide X comprend un composant (c) : huile qui est solide à 20 °C, et le rapport massique du composant (b) au composant (c) (b/c) est inférieur ou égal à 8.

5. Film de revêtement pour la peau humaine selon la revendication 2 ou la revendication 3, dans lequel la composition externe A comprend le composant (c) : huile qui est solide à 20 °C, et le rapport massique du composant (b) au composant (c), (b/c), dans la composition externe A est inférieur ou égal à 8.

6. Film de revêtement pour la peau humaine selon l'une quelconque des revendications 1 à 5, dans lequel la perméabilité à la vapeur, mesurée selon la méthode JIS Z2080 (1976), est comprise entre 300 et 3 000 g/m$^2$/24 h.

7. Film de revêtement pour la peau humaine selon l'une quelconque des revendications 1 à 6, qui est appliqué pendant 5 heures ou plus, à une perméabilité à la vapeur comprise entre 300 et 3 000 g/m$^2$/24 h.

8. Procédé non thérapeutique pour produire un film de revêtement pour l'être humain, dans lequel un film de revêtement présentant une perméabilité à la vapeur comprise entre 300 et 3 000 g/m$^2$/24 h est formé sur une surface cutanée, le procédé comprenant les étapes suivantes :

l'application d'une composition externe A sur la peau à un poids de base compris entre 0,5 et 2,3 mg/cm$^2$ ; et l'application d'une composition B sur la peau par un procédé d'électrofilage afin de former un assemblage de fibres présentant un poids de base compris entre 0,08 et 1,0 mg/cm$^2$,
dans lequel la composition externe A comprend le composant (a) suivant et peut éventuellement comprendre le composant (b) suivant, et présente une permittivité relative comprise entre 2 et 27, et
la composition B comprend de 40 % à 95 % en masse du composant (e) suivant et de 5 % à 35 % en masse du composant (f) suivant dans la composition B :

composant (a) : agent huileux dans un état liquide à 20 °C et qui présente une permittivité relative comprise entre 0,5 et 20 ;
composant (b) : un polyol dans un état liquide à 20 °C ;
composant (e) : une ou plusieurs substances volatiles choisies dans le groupe constitué d'alcools ; et
composant (f) : polymère insoluble dans l'eau en mesure de former un film de revêtement,
dans lequel le rapport entre le poids de base P de la préparation liquide A et le poids de base Q de l'assemblage de fibres (P/Q) est compris entre 2,5 ou plus et 10 ou moins :

dans lequel la permittivité relative de la préparation liquide A est une valeur totale obtenue en multipliant un rapport massique par les permittivités relatives de l'agent huileux dans un état liquide à 20 °C et du polyol dans un état liquide à 20 °C dans la préparation liquide, dans lequel, pour le calcul du rapport massique dans la préparation liquide A, un rapport massique de chaque composant par rapport à la masse de composants dans la préparation liquide A, à l'exclusion de composants volatils, est calculé ; dans lequel les composants volatils présentent une pression de vapeur à 20 °C comprise entre 1,33 kPa ou plus et 40,00 kPa ou moins, et dans lequel la permittivité relative est déterminée comme décrit dans la section « [Évaluation 5] Permittivité » de la spécification.

FIGURE 1

FIGURE 2

**EP 3 888 753 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004525272 A
- JP 2006095332 A
- JP 2007325934 A
- JP 2003506470 A
- JP 2004002356 A
- JP 2009536647 A
- JP 2012017297 A
- JP 2017078062 A
- JP 2018087186 A
- JP 2018108991 A